# EUROPEAN PATENT SPECIFICATION

(11) **EP 1 339 419 B1**
(45) Date of publication and mention of the grant of the patent: **07.02.2007**
(21) Application number: 01976574.2
(22) Date of filing: 29.10.2001
(51) Int. Cl.: A61K 35/74, A61P 1/02, A61P 1/04, A61K 31/70, A61K 31/7048, A61K 31/415

(54) **USE OF AGENTS ACTIVE AGAINST CANDIDA IN THE TREATMENT OF DISORDERS OF THE ORAL AND INTESTINAL MUCOSA**
VERWENDUNG VON GEGEN CANDIDA WIRKSAMEN MITTELN ZUR BEHANDLUNG VON STÖRUNGEN DER ORALEN UND INTESTINALEN MUCOSA
UTILISATION D'AGENTS ACTIFS CONTRE LE CANDIDA DANS LE TRAITEMENT DE TROUBLES DE LA MUQUEUSE BUCCALE ET INTESTINALE

(30) Priority: 02.11.2000 IT MI20002369
(43) Date of publication of application: 03.09.2003
(73) Proprietor: Proge Farm S.r.l., 28100 Novara (IT)
(72) Inventor: DONDI, Giancarla, I-28065 Cerano (IT)
(74) Representative: Gislon, Gabriele
(86) International application number: PCT/IB2001/002036
(87) International publication number: WO 2002/036137

(56) References cited:
- EP-A- 0 904 784
- WO-A-00/41707
- WO-A-94/02485
- WO-A-94/18206
- FR-M- 6 855
- O'MAHONY L. ET AL.: "Immunologic response to a novel probiotic organism in patients with active Crohn's disease." GASTROENTEROLOGY, vol. 118, no. 4, Suppl. 2, Part 1, April 2000 (2000-04), page AGA A853 XP001056680
- DUNNE C. ET AL.: "Probiotics: from myth to reality. Demonstration of functionality in animal models of disease and in human clinical trials." ANTONIE VAN LEEUWENHOEK, vol. 76, no. 1-4, July 1999 (1999-07), pages 279-292, XP001015705
- KENNEDY R.J. ET AL.: "Promotion of a favorable gut flora in inflammatory bowel disease" JOURNAL OF PARENTERAL AND ENTERAL NUTRITION, vol. 24, no. 3, May 2000 (2000-05), pages 189--195, XP001056663
- WENZEL P.: "Ökologie der Darmflora - ein Stiefkind der Medizin ?" THERAPIEWOCHE, vol. 44, no. 26, 1994, pages 1504-1506, XP001056674

## Description

### FIELD OF THE INVENTION

The present invention relates to the use of Lactobacilli activated against Candida for the prevention and/or treatment of some specific disorders of the intestinal mucosa.

More specifically, the invention relates to the use of Lactobacilli activated against Candida for the prevention and/or treatment of ulcerative colitis and Crohn's disease.

### TECHNICAL BACKGROUND

Ulcerative colitis and Crohn's disease are serious inflammatory pathologies affecting the intestinal mucosa.

The two pathologies have common aspects from the epidemiological, etiopathogenetic, anatomopathologic and clinical point of view, at least in the clinical pictures that involve the same intestinal segments and in the first phases of the disease; in full-blown cases it is almost always possible to make a correct differential diagnosis. For this reason the two diseases are usually classified under the common denomination of IBD "Inflammatory Bowel Disease".

The intestine affected by ulcerative colitis and/or Crohn's disease is ulcerative, hyperemic and generally hemorrhagic; distinctive features of Crohn's disease comprise the involvement of the ileum and the colon (whereas ulcerative colitis is restricted only to the colon and particularly to the rectum), the segmentary tendency of the lesions and their transmural involvement, the presence of granulomatous reactions which at times induce the formation of palpable masses and the mesenteric and lymph node involvement.

The main symptoms of the affected subject are diarrhoea often accompanied by loss of blood and abdominal pain, at times complicated by fever and weight loss. In the clinical pictures presented complications due to fibroses of the lesions, stenoses of the lumen, fissuration and intestinal fistulae can be observed.

The etiology of ulcerative colitis and Crohn's disease is not known; genetic, immunological, psychological and dietary factors have been hypothesized as causal agents. In fact, these diseases often have familiar incidence and are frequently accompanied by the formation of antibodies against the cells of the intestinal mucosa and by modifications of cellulo-mediated immunity; affected subjects often show clear neurotic signs of an anxious-depressive nature.

However, to date none of these factors seems to play a decisive role and the origin of the diseases remains unknown.

Consequently there are no causal therapies and treatments currently used, the same for ulcerative colitis and Crohn's disease, are mainly of the pathogenetic type aiming to control the inflammatory component and/or the immunity response; in particular, cortisone substances such as prednisolone are normally administered or, alternatively, non-cortisone anti-inflammatories such as mesalazine, hence treatments that are therefore palliatives, limited to reducing inflammation of the mucosa of the colon and unable to act either on the causes or the progression of the disease.

The patient affected by ulcerative colitis or Crohn's disease is therefore normally a patient subjected to chronic treatment, as the diseases are not adequately combated and the symptoms recur when treatment is stopped.

New pharmacological approaches are based on immunosuppressing agents which, as is known, cause serious collateral effects and still today do not offer definite results.

In some cases, the administration of bacteria to restore the bacterial flora, such as acidophilus, bifidus or saccharomyces boulardi help to ease the symptoms, improving the general condition of the intestine, although neither this type of treatment nor others currently available are truly effective in the treatment of the above diseases.

It is also known that Candida, generally Candida albicans, is an extremely widespread fungus and habitual saprophyte of the intestine, although not only, capable of causing diarrhoea in particular conditions (Bull. Soc. Path. Ex. 82, 1989,451-457) probably following its abnormal growth favoured, in specific situations, by specific intestinal disorders.

It has now been observed that the administration of specific agents active against Candida, such as specific strains of Lactobacilli having specifically induced activity against Candida, leads to complete remission of the aforesaid diseases and also permits recurrence to be prevented.

It has in fact been noted that some specific Lactobacilli, in particular Lactobacilli activated against Candida, are an excellent therapeutic treatment for ulcerative colitis and Crohn's disease.

It has thus been hypothesized that this fungus, Candida, plays an important role in the development of diseases affecting intestinal mucosa, such as ulcerative colitis and Crohn's disease, causing serious lesions of the mucosa in subjects prone to these pathologies.

To date no correlation between Candida and these diseases, in particular ulcerative colitis and Crohn's disease, had been hypothesized, and it is easily understood why this discovery is therefore extremely important for the purposes of potential radical treatment of said pathologies.

In particular, it has been found that the administration of agents active against Candida leads to a regression of the aforesaid diseases, up until their complete remission.

### DETAILED DESCRIPTION OF THE INVENTION

Therefore, the present invention relates, according to one of its aspects, to the use of Lactobacilli activated against Candida to prepare medicaments for the prevention and/or treatment of ulcerative colitis and Crohn's disease.

According to an advantageous aspect, the invention relates to the use of Lactobacilli modified by means of a method to induce activity against Candida, which are hence particularly effective.

This method to induce activity is described in detail and claimed in EP 0 949 330 (in the name of the present applicant) and is implemented with a technique according to which the addition of a pathogenic microorganism of Lactobacilli, or merely of an aliquot of culture supernatant of this in cultures, induces in these a powerful inhibiting activity towards said pathogen.

Candida is one of the pathogens towards which it has been possible to activate some strains of Lactobacilli, thus obtaining strains, hereinafter simply called "activated Lactobacilli", capable of suppressing Candida.

In particular, the patent describes a strain, already endowed with its own activity and also activated against Candida, which was isolated and filed in accordance with the Budapest Treaty. *Lactobacillus gasseri P-17632.*

Another strain endowed with activity against Candida is Lactobacillus *salivarius I* 1794.

One of the preferred objects of the present invention is hence the use of *Lactobacillus gasseri P-17632* and/or *Lactobacillus salivarius I 1794* to prepare medicaments intended for the treatment and/or prevention of ulcerative colitis and Crohn's disease.

In the treatment of ulcerative colitis and Crohn's disease, once administered orally, said activated Lactobacilli perform their action by colonizing the intestine, progressively eliminating the pathogens towards which they are activated, in the case in hand Candida, and preventing reinfection.

Other useful agents to be used in combination with the activated Lactobacilli according to the invention are orally active antimycotics of the convention type, providing they are effective against Candida, such as nistatine, amphotericine B, miconazole, ketoconazole, econazole, griseofulvine, fenticonazole, etc., hereinafter simply called "Conventional antimycotics".

It is understood that when wishing to use the above conventional antimycotics, their salts and/or prodrugs such as any esters, can also conveniently be used, providing these are pharmaceutically acceptable.

The active agents according to the invention are administered alone or in combination with one another and/or with other active substances and/or with pharmaceutically acceptable excipients and carriers.

It is important to note that different times are required for conventional antimycotics and Lactobacilli activated to take effect; in particular, whereas antimycotics act rapidly on the pathogen, the activity of the activated Lactobacilli takes longer, although offering the advantage of opposing any recurrence of the diseases thanks to colonization of the mucosae.

Consequently, although more time is required to attain the therapeutic result, the use of activated Lactobacilli is preferable, above all due to the absence of side effects and also because it offers more guarantee of success of the treatment, "watching over" the mucosa and obstructing reinstatement of the pathogen, hence preventing any relapses.

According to the state of development of the disease, it may be advisable to associate more than one active principle, such as a fast acting conventional antimycotic and a Lactobacillus activated against Candida. In this way a dual effect in obtained, as the conventional antimycotic rapidly eliminates Candida from the intestinal mucosa and the intestine is simultaneously colonized by the activated Lactobacilli.

The two agents may therefore be administered simultaneously during an initial phase of the treatment of ulcerative colitis and Crohn's disease, or alternatively, they may be administered sequentially; in other words, the treatment could entail a first treatment with conventional antimycotics and a second treatment based on activated Lactobacilli.

Nonetheless, it is to be noted that co-administration of the two agents is preferable, although not essential, and that it may be sufficient and/or preferable to administer solely activated Lactobacilli.

For the use according to the invention, conventional antimycotics are administered at doses officially recognized as effective; moreover, the weight and age of the subject and the severity of the disease shall also be taken into account.

The amount of activated Lactobacilli to be administered may instead vary within a vast range, as said Lactobacilli are totally without undesirable side effects.

The Lactobacilli activated against Candida may be administered in the range of 1 million to 100 billion live cells (UFC) per day, preferably in the form of pharmaceutical compositions.

Whether they are administered alone or in combination with one another other, the active ingredients can be administered in unit dosage forms, possibly together with appropriate excipients and carriers. The unit dosage forms shall preferably contain an effective amount of conventional antimycotic and/or a amount of activated Lactobacilli ranging from 1 million to 100 billion live cells (UFC), advantageously between 10 million and 10 billion, such as between 100 million and 1 billion live cells.

Advantageously, the compositions that must reach the intestine to perform the desired therapeutic effect are administered orally; nonetheless, should this be deemed appropriate and consented by the pathology, these compositions may be administered in other ways, such as by enema.

The unit forms of administration vary according to the active agents employed and comprise, for example, solid forms such as tablets, gelatine capsules, powders and grains or liquid forms such as solutions and suspensions.

For the administration of activated Lactobacilli formulations obtained, for example, from lyophilised powder are preferable, such as: powders for oral suspension, grains for sachets, capsules (hard or soft), tablets, suspensions, for example oily suspensions.

Other appropriate dosage forms may be envisaged, according to the agent utilized and the type of treatment to be administered.

Formulations can be prepared according to well known techniques, such as those described in Remington's Pharmaceutical Sciences, by E. W. Martin (Mack Publ. Co. Easton PA) and, in addition to the active ingredients, comprise the substances normally employed for this purpose in the pharmaceutical field and well known to the expert in the branch, as appropriate excipients and/or carriers.

The active ingredients may also be microencapsulated according to known techniques or be in the form of inclusion complexes such as cyclodextrin or similar.

The compositions are preferably administered from 1 to 3 times per day, advantageously 1 or 2 times per day, if possible on an empty stomach.

## Claims

1. Use of at least one Lactobacillus activated against Candida for the preparation of medicaments for the prevention and/or treatment of ulcerative colitis and Crohn's disease

2. Use as claimed in claim 1, wherein said Lactobacillus is chosen from *Lactobacillus gasseri P- 17632 and Lactobacillus salivarius I 1794.*

3. Use as claimed in claims 1 or 2, wherein said Lactobacillus is administered in oral pharmaceutical compositions.

4. Use as claimed in claim 3 , wherein said compositions comprise 1 million to 100 billion live cells.

5. Use as claimed in claims 1 to 4, for the treatment of ulcerative colitis and Crohn's disease, wherein said at least one Lactobacillus activated against Candida is administered in combination with at least one active agent which is an orally active conventional antimycotic.

6. Use as claimed in claim 5, wherein said conventional antimycotic is chosen from nistatine, amphotericine B, miconazole, ketoconazole, econazole, griseofulvine, fenticonazale, and their acceptable pharmaceutical salts.

## Patentansprüche

1. Verwendung von zumindest einem gegen Candida aktivierten *Lactobacillus-*Stamm zur Herstellung von Arzneimitteln zur Vorbeugung und/oder Behandlung von Colitis ulcerosa und Crohn'sche Krankheit.

2. Die Verwendung gemäß Anspruch 1, wobei der *Lactobacillus-Stamm* ausgewählt ist aus *Lactobacillus gasseri P-17632* und *Lactobacillus salivarius I* 1794.

3. Die Verwendung gemäß Anspruch 1 oder 2, wobei der *Lactobacillus-*Stamm als orale pharmazeutische Zusammensetzungen verabreicht wird.

4. Die Verwendung gemäß Anspruch 3, wobei die Zusammensetzungen 1 Million bis 100 Milliarden lebende Zellen umfassen.

5. Die Verwendung gemäß den Ansprüchen 1 bis 4, zur Behandlung von Colitis ulcerosa und Crohn'sche Krankheit, wobei der zumindest eine gegen Candida aktivierte *Lactobacillus*-Stamm in Kombination mit zumindest einem Wirkstoff verabreicht wird, der ein herkömmliches oral aktives Antimykotikum darstellt.

6. Die Verwendung gemäß Anspruch 5, wobei das herkömmliche Antimykotikum ausgewählt ist aus der Gruppe bestehend aus Nistatin, Amphotericin B, Miconazol, Ketoconazol, Econazol, Griseofulvin, Fenticonazol und pharmazeutisch annehmbare Salze davon.

## Revendications

1. Utilisation d'au moins un Lactobacillus activé contre le Candida pour la préparation de médicaments pour la prévention et/ou le traitement de la recto-colite hémorragique et de la maladie de Crohn.

2. Utilisation selon la revendication 1, dans laquelle ledit Lactobacillus est choisi parmi *Lactobacillus gasseri* P-17632 et *Lactobacillus salivarius I 1794.*

3. Utilisation selon la revendication 1 ou 2, dans laquelle ledit Lactobacillus est administré dans des compositions pharmaceutiques par voie orale.

4. Utilisation selon la revendication 3, dans laquelle lesdites compositions comprennent 1 million à 100 milliards de cellules vivantes.

5. Utilisation selon les revendications 1 à 4, pour le traitement de la recto-colite hémorragique et de la maladie de Crohn, dans laquelle ledit au moins un Lactobacillus activé contre le Candida est administré en combinaison à au moins un agent actif qui est un anti-fongique conventionnel actif par voie orale.

6. Utilisation selon la revendication 5, dans laquelle ledit anti-fongique conventionnel est choisi parmi la nistatine, l'amphotéricine B, le miconazole, le kétoconazole, l'éconazole, la griséofulvine, le fenticonazole, et leurs sels pharmaceutiquement acceptables.
